# EUROPEAN PATENT APPLICATION

(11) **EP 3 059 589 A1**
(43) Date of publication of application: **24.08.2016**
(21) Application number: 14854622.9
(22) Date of filing: 17.10.2014
(51) Int. Cl.: G01N 33/543, A61K 41/00, A61K 47/48, A61K 49/00, B22F 1/00, B22F 1/02

(54) **MULTIFUNCTIONAL METAL NANOSTRUCTURE AND METHOD FOR PRODUCING SAME**

(30) Priority: 18.10.2013 US 201314057609
(71) Applicant: IMRA America, Inc., Ann Arbor, MI 48105 (US); Japanese Foundation For Cancer Research, Tokyo 135-8550 (JP)
(72) Inventor: ICHIKAWA, Yuki, Kariya-shi Aichi 448-8650 (JP); SHIBA, Kiyotaka, Tokyo 135-8550 (JP)
(74) Representative: Schön, Christoph
(86) International application number: PCT/JP2014/077633
(87) International publication number: WO 2015/056766

(57) **Abstract**

Provide is a stable metallic nanostructure that causes no aggregation when surface-modified with biomolecule-reactive functional molecules. 30 to 90% of the surface of the metallic nanostructure is covered with at least one or more types of colloid-stabilizing functional molecules. Furthermore, the metallic nanostructure is covered with one or more types of biologically functional molecules.

## Description

### Field of the Invention

The present invention relates to a medical multifunctional metallic nanostructure for use in the diagnosis or treatment of disease. Specifically, the present invention relates to a method for covering the surface of a metallic nanostructure with a plurality of functionalizing molecules to prepare a stable colloidal dispersion, a multifunctional metallic nanostructure obtained by the method, and a product comprising the multifunctional metallic nanostructure.

### Description of the Related Art

So-called nanotechnology using metallic nanostructures such as metal nanoparticles or nanorods has become important in the research or industrial field in recent years. Particularly, in the medical or diagnostic field, such metallic nanostructures are surface-bound with functionalizing molecules such as biomolecules (e.g., peptides or nucleic acids), biocompatible polymers, or fluorescent molecules and utilized in, for example, the detection of disease.

Particularly, gold nanostructures, which contain gold as a metallic component, have been extensively developed, because gold is a stable substance with low toxicity. Colorimetric sensors or sensors utilizing surface plasmon resonance asked on the optical properties of the gold nanostructures are used in various tests, for example, home pregnancy test kits.

The metallic nanostructures are also surface-coated with particular molecules and used as probes for dark-field microscopes or electron microscopes. A further attempt has been made to coat the surfaces of the gold nanostructures with targeting molecules that recognize particular cells (e.g., cancer cells) and with therapeutic drugs and use the resulting nanostructures as carriers for treatment.

The surfaces of these metallic nanostructures are usually functionalized by: mixing functional molecules with a colloidal solution containing core metallic nanostructures dispersed in a liquid medium such as water; and modifying the surfaces of the metallic nanostructures through the binding reaction between the metal nanoparticles and the functional molecules that occurs either spontaneously or by external stimulus such as pH change or temperature change.

### Citation List

### Patent Literature

Patent Literature 1: US Patent Application Publication No. 2012/0225021 Non-Patent Literature
Non-Patent Literature 1: G. Luo et al., International Journal of Pharmaceutics, 2010, Vol. 385, pp. 150-156;
Non-Patent Literature 2: G. F. Paciotti et al., Drug Delivery, 2004, Vol. 11, pp. 169-183
Non-Patent Literature 3: K. A. Kelly et al., PLOS Medicine, 2008, Vol.5, Issue 4, e45
Non-Patent Literature 4: S.J. Shin, et al., PNAS, 2013, Vol. 110, No. 48, pp.19414-19419

### Summary of the Invention

### Problems to be Solved by the Invention

The mixing of the colloidal solution of metallic nanostructures with biomolecule-reactive functional molecules upon functionalization of the metallic nanostructures may destabilize the colloidal state and induce the aggregation of the metallic nanostructures. Once the nanostructures aggregate, they can rarely be redispersed. Particularly, molecules having a charged functional group, for example, peptides having a molecular weight of approximately 3000 or lower, frequently cause the aggregation of the metallic nanostructures. Unfortunately, such metallic nanostructures are difficult to utilize in biotechnological or medical use.

The following approaches are used for circumventing these problems: the first method involves initially binding one end of biocompatible polymers such as polyethylene glycol (PEG) to the surface of each nanostructure to modify the whole surface of the nanostructure. Since interparticle repulsion occurs due to steric hindrance by the polymers, the colloid is stabilized and prevented from aggregating.

After the surface modification with the polymers, functionalizing molecules of interest are bound to the other ends of the polymers through chemical reaction. In short, this method binds the functionalizing molecules of interest to the outer polymer layer of each nanostructure via the polymers (Non-Patent Literature 1).

This method, however, produces undesired increases in the overall size of the surface-modified nanostructure due to the sequential layering of its outer surface. In addition, the method involves the chemical binding of the functionalizing molecules and therefore requires introducing functional groups for binding in advance to both of the polymers and the functionalizing molecules. This disadvantageously results in the complicated synthesis of these molecules and large cost.

The second method involves adjusting the pH of the colloidal solution according to particular proteins or peptides for surface modification and surface-modifying nanostructures under the prescribed pH (Non-Patent Literature 2).

This method, however, requires performing the reaction according to the optimum pH specific for the proteins or the peptides. The optimum pH must therefore be determined for individual proteins or peptides. Thus, this approach fails to establish a general surface modification method and requires a time for pH optimization on a protein or peptide basis.

Alternatively, Patent Literature 1 discloses a method for modifying functional molecules by the adjustment of surface coverage, and stabilized colloidal nanoparticles obtained by modification. This literature suggests the application of these nanoparticles to biological or medical use. Nonetheless, Patent Literature 1 only discloses stabilized colloidal nanoparticles in which polyethylene glycol was actually bound to a gold nanoparticle surface, and no mention is made therein about the applied technology of modification using molecules, such as peptides or aptamers, which are capable of specifically binding to biomolecules.

Thus, the colloidal particles described in Patent Literature 1 were stabilized colloidal nanoparticles, but did not undergo optimization for medical or diagnostic use through their binding to the molecules such as peptides or aptamers.

Sensitivity and accuracy are required in order to detect biomolecule such as a specific protein or the like for treatment or diagnostic purpose. In this regard, it is necessary to modify the metal nanostructure by a functional molecule which bonds to biomolecules such as peptides or aptamers at high density. Moreover, since the biomolecules are diverse and therefore the functional molecules which bond thereto are also diverse. Therefore, it is necessary to further optimize the surface modification method to be applied to various biomolecules and to be used for treatment or diagnostic purpose.

### Solution to Problems

The multifunctional metallic nanostructure of the present invention comprises a metallic nanostructure having a surface covered with: at least one or more types of colloid-stabilizing functional molecules which cover 30 to 90% of the surface of the metallic nanostructure; and at least one or more types of biologically functional molecules having a terminal amino acid.

The colloid-stabilizing functional molecules prevent the metallic nanostructure from aggregating, while the biomolecule-reactive biologically functional molecules bind to their target molecules. Thus, the multifunctional metallic nanostructure can be applied as a material for diagnosis or treatment.

In this context, it is important that the colloid-stabilizing functional molecules should not cover the whole region of the metallic nanostructure surface but should partially cover the metallic nanostructure surface. This covering with the colloid-stabilizing functional molecules secures the dispersibility of the metallic nanostructure in an aqueous solution, while the partial covering allows the biologically functional molecules to bind to the gaps or the non-covered region between the colloid-stabilizing functional molecules.

If the coverage with the colloid-stabilizing functional molecules is less than 30%, the resulting metallic nanostructure easily aggregates and fails to produce a stable colloid. If the coverage with the colloid-stabilizing functional molecules exceeds 90%, the biologically functional molecules cover only a small region. The resulting metallic nanostructure is low reactive with biomolecules.

As the biologically functional molecules having a terminal amino acid, antibody, and various peptides such as synthetic peptide which bonds to specific molecules, peptide hormone or the like, are included. Furthermore, it may include molecules of peptide nucleic acid (PNA) or nucleic acids bound with linkers, and the linkers are not particularly limited as long as they are compounds including amino group. By bonding these molecules as the biologically functional molecules to the multifunctional metallic nanostructure, a wide range of application to diagnosis, treatment, research fields, or the like can be expected.

In the multifunctional metallic nanostructure of the present invention, the colloid-stabilizing functional molecules include a compound represented by a following general formula (1):

[Formula 1] -(CH₂-CH₂-O)ₙ- (1)

wherein n represents an integer of 1 or larger.

Such a metallic nanostructure comprising the compound is stabilized as a colloid.

In the multifunctional metallic nanostructure of the present invention, the compound of the general formula (1) is polyethylene glycol or a derivative thereof.

Since polyethylene glycol is highly biocompatible, it can be used for the multifunctional metallic nanostructure together with therapeutic drugs for particular targets (e.g., cancer cells) and thereby administered to an organism.

In the multifunctional metallic nanostructure of the present invention, the colloid-stabilizing functional molecules each have a thiol group or a disulfide group at least at one end thereof.

Such colloid-stabilizing functional molecules each having a functional group including a thiol (- SH) or a disulfide (- S - S -) at one end are capable of easily binding to a metallic base material. Thus, the metallic nanostructure can be reliably covered with the colloid-stabilizing functional molecules.

In the multifunctional metallic nanostructure of the present invention, the colloid-stabilizing functional molecules each have a functional group including a thiol (-SH) or a disulfide (- S - S -) at one end and at least any one of a methoxy group, an amino group, a carboxy group, an acyl group, an azo group, and a carbonyl group at the other end.

Such colloid-stabilizing functional molecules each having any one of a methoxy group, an amino group, a carboxy group, an acyl group, an azo group, and a carbonyl group are also capable of binding to peptides serving as the biologically functional molecules. The surface region to which the biologically functional molecules can bind is therefore expanded.

The multifunctional metallic nanostructure of the present invention is a metal nanoparticle which is a noble metal nanoparticle or a noble metal-containing alloy nanoparticle.

Such a noble metal nanoparticle, i.e. platinum or the like, or noble metal-containing alloy serving as the metallic nanostructure has a large scattering coefficient for radiation and as such, can be used as, for example, an X-ray or particle beam contrast agent.

In the multifunctional metallic nanostructure of the present invention, the metal nanoparticle is a gold nanoparticle or a gold-containing alloy nanoparticle.

Among noble metals, gold is stable and has already been used as a carrier in diagnosis or treatment. In addition, a wide range of use has already been established for the gold nanoparticle. Such nanoparticles can be accumulated in target cells such as cancer cells via the biologically functional molecules and kill the target cells by means of heat generated using electromagnetic wave irradiation.

In the multifunctional metallic nanostructure of the present invention, the biologically functional molecules each comprise at least peptide.

Peptides having molecular weight of 200 or more to 10000 or less which bond to target moleculars are able to efficiently cover the metallic nanostructure. Therefore, it is able to detect the target moleculars with high sensitivity, and high effect can be expected when using for treatment.

The present invention further provides a dispersion of the multifunctional metallic nanostructure dispersed in a liquid.

The multifunctional metallic nanostructure of the present invention has a surface covered with the colloid-stabilizing functional molecules and is therefore very stably dispersed in a liquid. Thus, the multifunctional metallic nanostructure of the present invention is very easy to handle. The multifunctional metallic nanostructure of the present invention is also bound with the biologically functional molecules and as such, can be provided in a ready-to-use form at the scene of diagnosis or treatment.

The present invention further provides a freeze-dried product comprising the multifunctional metallic nanostructure, wherein the dispersion is frozen.

Such a freeze-dried product can be stored for a long period and secure transportation stability. The freeze-dried product can be supplied as a stable product even in a state bound with the biologically functional molecules such as peptides.

The composition for diagnosis and/or treatment of the present invention comprises the multifunctional metallic nanostructure.

The multifunctional metallic nanostructure of the present invention, which is a metallic nanostructure bound with biologically functional molecules, can be accumulated in a desired organ, affected area, or the like and may be used in a contrast medium or thermotherapy. Also, the multifunctional metallic nanostructure of the present invention may be bound with dyes such as fluorescent dyes and thereby used as a so-called imaging agent to detect cancer cells or the like.

The multifunctional metallic nanostructure of the present invention can also be used as a carrier for anticancer agents. Specifically, the multifunctional metallic nanostructure bound with anticancer agents or cell growth inhibitors together with the biologically functional molecules can be accumulated in target cells and permits treatment with few adverse reactions.

The method for manufacturing a multifunctional metallic nanostructure according to the present invention comprises the steps of: providing a metallic nanostructure dispersed in water or an electrolyte solution; covering 30 to 90% of the surface of the metallic nanostructure with colloid-stabilizing functional molecules, by monitoring the amount of surface covering of the metallic nanostructure with the measurement of a physical quantity; and then covering the surface of the metallic nanostructure with one or more types of biologically functional molecules.

The amount of surface covering of the metallic nanostructure can be monitored by the measurement of a physical quantity. Thus, the surface of the metallic nanostructure can be first covered with colloid-stabilizing functional molecules with the coverage adjusted and next, also covered with biologically functional molecules under monitoring of the coverage. The surface of the metallic nanostructure can therefore be covered with the colloid-stabilizing functional molecules and the biologically functional molecules at the optimum ratio.

Furthermore, it is possible to obtain the surface covering ratio of the metallic nanostructure according to a predetermined covering condition in advance, and to cover the colloid-stabilizing functional molecules based on the surface covering ratio. Especially, in a case of using specific colloid-stabilizing functional molecules such as PEG or the like, it is able to cover the surface of the metallic nanostructure with the desired covering ratio with high reproducibility by covering according to the determined condition.

In the method for manufacturing a multifunctional metallic nanostructure according to the present invention, a dispersion of the metallic nanostructure dispersed in water or an electrolytic solution has an electric conductivity of approximately 25 µS/cm or lower.

This is because impurity ions might impair the surface activity of the gold nanoparticle in the surface covering step.

In the method for manufacturing a multifunctional metallic nanostructure according to the present invention, the one or more types of biologically functional molecules are N types (wherein N represents an integer) of biologically functional molecules, the method further comprising individual steps of using the first biologically functional molecules to the (N - 1)th biologically functional molecules as the biologically functional molecules to each partially cover the surface of the metallic nanostructure in order so as not to occupy the whole of the effective surface area thereof, while adjusting the amount of surface covering of the metallic nanostructure in each of the individual steps by the measurement of a physical quantity, whereafter the metallic nanostructure is surface-covered with the Nth biologically functional molecules until an effective region on the surface of the metallic nanostructure becomes saturated.

The method for manufacturing a multifunctional metallic nanostructure according to the present invention can cover the metallic nanostructure surface with even plural types of biologically functional molecules with the amount of covering adjusted and therefore achieves covering at their respective optimum ratios.

The method for manufacturing a multifunctional metallic nanostructure according to the present invention further comprises the step of removing redundant molecules unbound with the metallic nanostructure after each of the individual steps.

Such a manufacturing method further comprising the step of removing redundant molecules enables the coverage of the metallic nanostructure surface to be measured more accurately.

In the method for manufacturing a multifunctional metallic nanostructure according to the present invention, the step of removing redundant molecules involves centrifugation or dialysis.

Such redundant molecules can be conveniently removed by centrifugation. Alternatively, the redundant molecules can be removed by dialysis to thereby manufacture a drug safely administrable as a contrast medium or a therapeutic drug.

The present invention further provides a kit for manufacturing the multifunctional metallic nanostructure of the present invention, comprising: a metallic nanostructure partially covered with at least one or more types of colloid-stabilizing functional molecules; and a buffer solution for covering with biologically functional molecules.

Such partial covering with the colloid-stabilizing functional molecules allows a researcher to appropriately cover the metallic nanostructure surface with desired biologically functional molecules and thereby prepare a reagent according to his or her purpose of research or diagnosis.

### Brief Description of the Drawings

FIG. 1 schematically shows the multifunctional metallic nanostructure of the present invention;
FIG. 2 shows an increase amount of a hydrodynamic particle radius in mixed solutions differing in a ratio of number of molecules of PEG: number of gold nanoparticles;
FIG. 3A shows a modification ratio of a surface of gold nanoparticles which differs in ratio of number of molecules of PEG: number of gold nanoparticles;
FIG. 3B shows a suppression of aggregation of gold nanoparticles covered by PEG;
FIG. 4A to FIG. 4H are images of cell staining using the multifunctional gold nanoparticles modified with EpCAM-binding peptides;
FIG. 5A to FIG. 5C are images of cell staining using the multifunctional gold nanoparticles modified with plectin binding peptides; and
FIG. 6 is a diagram showing fluorescence intensity of fluorescent mark multifunctional gold nanoparticles.

### Detailed Description of the Preferred Embodiments

Any molecule that is effective for preventing colloidal particles from aggregating may be used as the colloid-stabilizing functional molecules of the present invention.

The surface covering of particles with polymers keeps the particles at some distance from each other due to steric hindrance by the covering molecules and therefore substantially prevents the particles from aggregating. Thus, any polymer capable of covering metal surface may be used in the present invention.

Examples of such colloid-stabilizing functional molecules include polyethylene glycol (PEG), polyacrylamide, polysaccharide, polydecyl methacrylate, polymethacrylate, polystyrene, polycaprolactone (PCL), polylactic acid (PLA), poly(lactic-co-glycolic acid) (PLGA), polyglycolic acid (PGA), polyhydroxybutyrate (PHB), macromolecular hydrocarbon, and their derivatives and copolymers. Further examples of the colloid-stabilizing functional molecules include dendrimers, aptamers, DNAs, RNAs, peptides, antibodies, and proteins (e.g., albumin).

Although, generally, aptamer or protein molecules cause aggregation, certain aptamers or proteins induces only steric hindrance without causing aggregation. Such aptamers or proteins can act as the colloid-stabilizing functional molecules.

Alternatively, a surfactant (e.g., sodium dodecyl sulfate (SDS), lithium dodecyl sulfate (LDS), Tween 20, Tween 80, Triton-X100, and cholic acids), polyvinylpyrrolidone (PVP), or the like may be used.

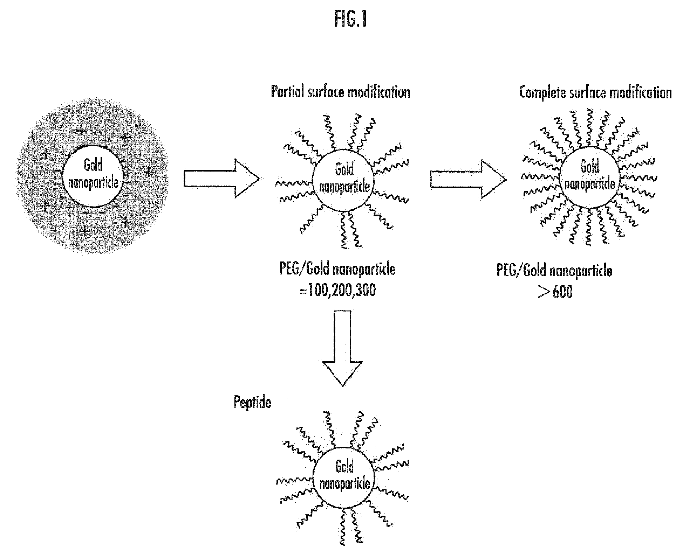

For covering with biologically functional molecules, it is important to adjust the amount of covering with the colloid-stabilizing functional molecules so that the metal nanoparticle surface is partially covered therewith, as schematically shown in FIG. 1.

As shown in FIG. 1, a colloid is stabilized when the particle surface is completely covered with the colloid-stabilizing functional molecules (PEG is taken as an example in FIG. 1). In this case, however, the biologically functional molecules (peptides are taken as an example in FIG. 1) have no space to enter the gaps between the colloid-stabilizing functional molecules. As a result, the biologically functional molecules (e.g., peptides) are hindered from binding thereto. Thus, for achieving these two factors, i.e., the colloid stabilization and the binding of the biologically functional molecules, it is important to partially cover the metal nanoparticle surface with the colloid-stabilizing functional molecules.

The term "binding" or "bond" used herein encompasses every binding pattern including covalent bonds, hydrogen bonds, ionic bonds, and van der Waals bonds.

PEG suitable for use in the present invention preferably has a molecular weight on the order of 500 to 100000, which however differs depending on the molecular weight of the biologically functional molecules to be bound as the second functional molecules.

Any biomolecule-reactive molecule may be used as the biologically functional molecules of the present invention. For example, nucleic acids or peptides, such as antibodies or aptamers, which are capable of specifically binding to particular molecules can be used. In the case of using nucleic acids, a linker having amino groups should be added as described above.

The peptides, for example, are expected to efficiently bind to the metallic nanostructure when having a molecular weight in the range of 200 or higher and 10000 or lower. The biologically functional molecules of the present invention, however, are not limited to the peptides having a molecular weight of 200 or higher and 10000 or lower. Various molecules, including antibodies having a molecular weight exceeding 100000, are possible. Various peptides such as synthetic peptides or peptide hormones capable of binding to particular molecules, and their derivatives are also possible biologically functional molecules of the present invention.

The metallic nanostructure bound with plural types of biologically functional molecules may be variously applied in diagnostic or therapeutic use. The multifunctional metallic nanostructure bound with, for example, fluorescent agents or dyes together with the antibodies or aptamers for binding to targets shows its power in diagnosis or treatment using an endoscope. Alternatively, the multifunctional metallic nanostructure bound with, for example, compounds such as anticancer agents together with the targeting molecules also permits treatment targeting particular cells.

For use in diagnosis or treatment, for example, peptides having affinity for a cancer stem cell surface marker EpCAM (epithelial cell adhesion molecule) are bound as the biologically functional molecules to the metallic nanostructure bound with the colloid-stabilizing functional molecules. Upon administration to an organism, this multifunctional metallic nanostructure binds to a cancer focus. This enables the cancer focus to be visualized via the gold nanocolloid and diagnosed using a diagnostic imaging apparatus for X-ray examination or the like.

Endoscopic muscularis dissection (EMD) or endoscopic submucosal dissection (ESD) is selected as the first choice for gastric mucosal cancer in endoscopic surgery, which has become significantly increasingly utilized in recent years. Also, endoscopic dissection (polypectomy) is widely practiced for polyps in the large intestine as general treatment. For these diagnostic or therapeutic procedures, the multifunctional metallic nanostructure further bound with fluorescent dyes can be administered to a wide surgical field under an endoscope and irradiated with fluorescence excitation laser to thereby make the cancer focus detectable as a fluorescent site. Consequently, a surgical dissection site can be determined.

The multifunctional metallic nanostructure of the present invention can be further utilized for therapeutic purposes by a method which involves administering the multifunctional metallic nanostructure and then exciting the metallic nanostructure by the application of some external physical energy such as electromagnetic wave (e.g., microwave or light) or ultrasound to locally apply heat to the affected area. Such energy excitation can be carried out using any energy level or energy level combination specific for the nanostructure, including energies such as electronic transition, lattice vibration, and vibration or rotation of the nanostructure. For example, gold nanoparticles have plasmon resonance attributed to the collective vibration mode of localized electrons. In this respect, the gold nanoparticles are selectively excited by irradiation with laser light with a wavelength corresponding to this resonance energy. As a result, the ambient temperature of the gold nanoparticles becomes high due to thermal energy converted through electron-lattice interaction and lattice-lattice interaction. Since cancer cells die at 42°C or higher, this nanostructure can be utilized in the so-called thermotherapy of cancer.

Alternatively, the multifunctional metallic nanostructure of the present invention may be further bound with anticancer agents and used in cancer treatment as a drug delivery system targeting cancer stem cells. In this case, the multifunctional metallic nanostructure is accumulated in cancer tissues, because their blood vessels of neovascularization are generally more vulnerable and more substance-permeable than capillary vessels of original tissues. The anticancer agents bound thereto, which have a given mass and low protein interaction, are relatively concentrated to prevent from reacting with cells or tissues other than the target site or being widely diffused in the body. The anticancer agents are therefore accumulated in the target site. Consequently, this approach can also be expected to be effective for suppressing adverse reactions or increasing anticancer drug efficacy. In order to allow the intracellularly taken-up multifunctional metallic nanostructure to release drugs into the cells, a substrate containing peptide-bonds cleaverage by intracellular protease (e.g., cathepsin) can be used as a linker that binds the drugs to the metallic nanostructure.

Moreover, when used for cancer diagnosis and treatment, since the blood vessels of neovascularization of cancer tissues are more substance-permeable than normal vessels, it is able to increase the delivery to the cancer tissues than to normal tissues by adjusting the size of the metal nanostructure, thereby enabling to develop a method with less adverse reactions and increased efficacy.

In addition to EpCAM, molecules such as HER2 (human EGFR-related 2), MUC1 (mucin core protein 1), FGFR2 (fibroblast growth factor receptor 2), CD44, CD59, CD133, CD81, VEGFR (vascular endothelial growth factor receptor), IGF-1R (insulin-like growth factor 1 receptor), EGFR (epidermal growth factor receptor), IL -10 receptor, IL-11 receptor, IL-4 receptor, PDGF (platelet-derived growth factor) receptor, chemokine receptor, E-cadherin, integrin, claudin, Fzd10, plectin, TAG-72, prestin, clusterin, nestin, selectin, tenascin C, and vimentin are known to be expressed in particular cancer cells or cancer stem cells. The metallic nanostructure of the present invention can be bound with, for example, antibodies or aptamers capable of binding to these molecules and thereby usefully used in diagnosis or treatment.

The technique of delivering particular nucleic acids into cells is necessary for the field of nucleic acid drugs. The multifunctional metallic nanostructure of the present invention can also be used as a carrier for this delivery system. Specifically, the metallic nanostructure of the present invention can be bound with siRNAs, shRNAs, microRNAs, or other nucleic acid molecules such as antisense nucleic acids or decoy nucleic acids either in themselves or via linkers and thereby usefully used in diagnosis or treatment by delivery into cells.

The biologically functional molecules each having a terminal amino acid can stably bind to the metallic nanostructure. The amino acid does not have to contain a thiol group, i.e., does not have to be cysteine.

A formulation using the multifunctional metallic nanostructure of the present invention can be provided as a dispersion or as a freeze-dried product. The formulation in a dispersion form can be ready to use. The freeze-dried product may be stored for a long period.

The present invention further provides a kit comprising: a metallic nanostructure partially covered with colloid-stabilizing functional molecules; and a buffer solution for the binding of biologically functional molecules. Use of the metallic nanostructure partially covered with the colloid-stabilizing functional molecules allows a user to bind desired biologically functional molecules to the metallic nanostructure used. Hereinafter, the present invention will be described in detail with reference to Examples.

### Examples

### [Example 1] Surface covering of metal nanoparticle

### (1) Partial surface covering of metal nanoparticle with first functional molecule (colloid-stabilizing functional molecule)

A colloidal solution of gold nanoparticles of approximately 15 nm, specifically, i-colloid Au15 (manufactured by IMRA America, Inc., USA), prepared by in-liquid laser ablation was provided as a colloidal solution of metal nanoparticles serving as a core for multifunctional metallic nanostructures and used as a precursor. The solution had a gold nanoparticle concentration of approximately 2.8 nM.

Lower amounts of impurity ions are more preferred for the total concentration of electrolytes contained in the colloid. Desirably, the colloidal solution has an electric conductivity of approximately 25 µS/cm or lower. A colloidal solution of chemically-synthesized gold nanoparticles prepared by, for example, a citrate reduction method generally widely used is rich in impurity ions such as reaction by-products and therefore has an electric conductivity from 200 µS/cm to 300 µS/cm or higher. Not only might these impurity ions impair the surface activity of the gold nanoparticles in the surface covering step described below, but also might the presence of impurity ions (electrolytes) in large amounts reduce the thickness of an electric double layer serving as a source of electrostatic repulsion applied to between the colloidal particles, resulting in problems such as particle aggregation in the molecular surface covering step.

Here, the first functional molecules (colloid-stabilizing functional molecules) used were thiolated methoxy-polyethylene glycol with a molecular weight of approximately 5000, specifically, mPEG-SH, 5k (manufactured by Creative PEGWorks, Creative Biotechnology LLC.), dissolved in deionized water.

First, the mixing ratio between the colloid-stabilizing functional molecules, i.e., PEG, and the gold nanoparticles suitable for the partial surface covering of the metal nanoparticles with PEG is determined.

Mixed solutions differing by degrees in the ratio between the gold nanoparticles and PEG are provided. Each mixed solution is well blended and then stilled for 24 hours. PEG binds to gold through a thiol-gold chemical bond formed on the gold nanoparticle surface.

The percentage at which PEG occupied the metal nanoparticle surface was estimated on the basis of changes in hydrodynamic particle radius in dynamic light scattering (DLS). Specifically, the particle size is measured using Zetasizer Nano ZS (manufactured by Malvern Instruments Ltd., UK). Provided that occupancy becomes 100% saturated at a value to which radial increment asymptotically approaches, a percentage approaching to this asymptote is defined as nanostructure surface coverage.

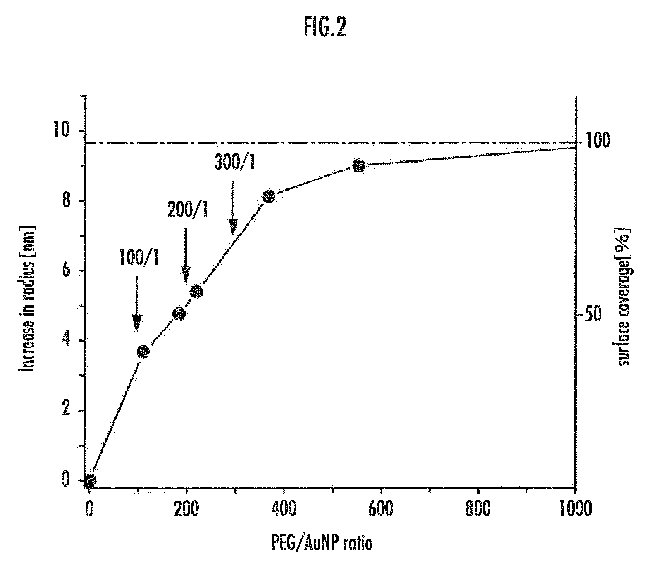

FIG. 2 shows increases in hydrodynamic particle radius measured by DLS in the mixed solutions differing in number of PEG molecules: number of gold nanoparticle ratio. As the ratio of PEG to the gold nanoparticles increases with respect to gold nanoparticles, the increment of the hydrodynamic particle radius asymptotically approaches to 10 nm. Accordingly, radial increment of 10 nm or near is confirmed as a saturated region close to 100% occupancy (shown in the right ordinate of FIG. 2). The state of partial surface covering with PEG shown in FIG. 1 is achieved in regions having a ratio of 600 or smaller between the number of the PEG molecules and the number of the gold nanoparticles at which the hydrodynamic particle radius shows a sharp increase in the graph of FIG. 2, for example, at points of 100/1,200/1, and 300/1 indicated by arrows in FIG. 2.

The covering of approximately 30% or more of the metal surface with the colloid-stabilizing functional molecules seems to be necessary for a stable dispersion without aggregation of the metallic nanostructures bound with the colloid-stabilizing functional molecules such as PEG molecules. In another experiment, colloids partially covered with mPEG-SH, 5k at these varying ratios (100/1, 200/1, and 300/1) were mixed with, for example, RAD peptide solutions, and discoloration attributed to particle aggregation was confirmed in the colloid having the 100/1 ratio corresponding to the coverage of approximately 30%. This suggests that approximately 30% or more of the colloidal particle surface should be covered.

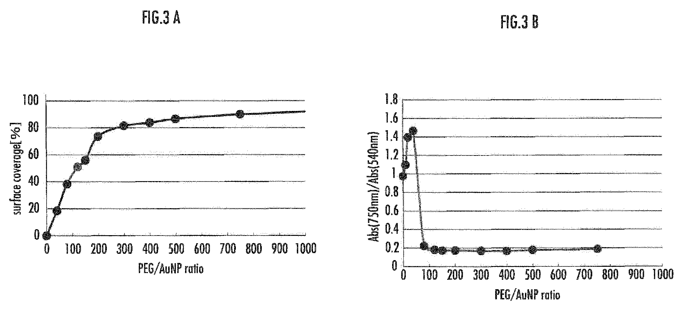

Next, it was confirmed that aggregation of the nanostructure was less likely to occur by covering of the colloid-stabilizing functional molecules. The gold nanoparticles were covered with PEG in the same manner as above while changing the value of ratio of number of PEG molecules and number of gold nanoparticle from 10/1 (PEG amount is 10 times) to 750/1 (PEG amount is 750 times). As shown in FIG. 3A, the surface modification ratio was approximately 38% when the number of PEG molecules was 80 times compared to the number of gold nanoparticles, and the surface modification ratio was approximately 74% when the number of PEG molecules was 200 times compared to the number of gold nanoparticles.

Next, the surface covered gold nanoparticles were suspended in 10% NaCl which is close to physiological salt concentration by changing the ratio of number of PEG molecules and the number of gold nanoparticles, and the aggregation of gold nanoparticles was measured by absorbancy (FIG. 3B). It was clear that the aggregation of gold nanoparticles was suppressed when covered by the number of PEG molecules being 80 times or more compared to the number of gold nanoparticles. Accordingly, if 40% or more of the surface of the metal nanostructure is covered, it is able to obtain a metal nanostructure in which the aggregation is suppressed.

### (2) Surface covering of metal nanoparticle with second functional molecule (biologically functional molecule)

Next, the binding of peptides as the second functional molecules (biologically functional molecules) will be shown as an example. The peptides used were EpCAM-binding peptides with fluorescent molecule, fluorescein isothiocyanate (FITC) bound at amino terminal KHLQCVRNICWSGGK. (SEQ ID NO: 1, hereinafter, referred to as Ep114). EpCAM is an antigen confirmed to be expressed on the surface of cancer cells.

Gold nanoparticles partially covered with colloid-stabilizing functional molecules PEG at number of PEG molecules : number of gold nanoparticle ratio values of 100/1, 200/1, and 300/1 were provided. Next, Ep114 peptide solutions are each concentration-adjusted so that the ratio of the number of the Ep114 peptides to the number of the gold nanoparticles finally becomes 2000 in mixed solutions. The Ep114 peptide solutions are added to the PEG/gold nanoparticle mixed solutions and mixed therewith. The biologically functional molecules thus added in excess can cover uncovered portions of the gold nanoparticles partially covered with the colloid-stabilizing functional molecules.

The resulting mixtures can be stilled for approximately 12 to 24 hours to bind the peptides to the PEG-bound gold nanoparticles.

After the completion of covering of the metal nanoparticles, redundant functional molecules can be removed using a routine method such as centrifugation or dialysis.

Here, each mixed solution after the covering with the Ep 114 peptides was placed in a centrifuge tube and centrifuged at 16,000 g at 4°C for 90 minutes. After removal of the supernatant, deionized water was added to the residue, and the resulting solution was washed by centrifugation again, followed by addition of a medium for cells. In this way, Ep 114/PEG/gold nanoparticle complexes dispersed in the medium for cells were obtained.

Needless to say, a user can appropriately select any solution in which the complexes are fmally dispersed, depending on the use purpose of the multifunctional metallic nanostructure.

### [Example 2] Cell staining using multifunctional metallic nanostructure covered with EpCAM-binding peptides

A colon cancer cell line HT29 was used to conduct a cellular uptake test. First, by using 96 holes type culture plate for spheroids, EZ-Sphere ^{™} (Asahi Glass Co., Ltd.), spheroid of HT29 cells was formed.

Specifically, HT29, 4x10 ⁵ cells were suspended in a culture medium in which 20ng / ml of human EGF (manufactured by Miltenyi Biotec, K.K.), 20ng / ml of human FGF-2 (manufactured by Miltenyi Biotec K.K.), 1/50 amount of B-27 supplement × 50 (manufactured by GIBCO), and 1/100 amount of Penicillin-Streptomycin Solution × 100 (manufactured by Wako Pure Chemical Industries, Ltd.) were added to 3ml of D-MEM / F-12 medium (Dulbecco's Modified Eagle Medium: Nutrient Mixture F-12 1: 1 Mixture, manufactured by GIBCO). The suspension was dispensed to each well of 200µl, and was cultured in CO₂ incubator at 37°C for 72 hours. Then, the supernatant portion 150µl which does not include spheroid was removed, and the spheroids in suspension 50µl which precipitated at the bottom was added with 50µl of solution of multifunctional metallic nanostructure covered with EpCAM-binding peptides (EP114 peptide, amino acid sequence shown in SEQ ID NO: 1) and peptides that does not bind to EpCAM (EP114 control peptide, amino acid sequence shown in SEQ ID NO: 2), and then reacted at 37°C for 1 hour.

Then, a part of the spheroids (approximately 20µl) and the gold particle-peptide complex were placed on a glass bottom dish (D110300, Matsunami Glass Ind., Ltd.), and irradiated laser of 488nm with 40 magnification objective lens by using an inverted confocal laser microscope (FLUOVIEW FV1000IX81 type, manufactured by Olympus Corporation), and observed using a filter for Alexa488.

In the multifunctional metal nanostructure covered with the EpCAM-binding peptides used or control peptides, the multifunctional metal nanoparticles were prepared and used according to the method described in Example 1. Specifically, gold nanoparticles were partially covered such that the ratio values of the number of PEG molecules : number of gold nanoparticles were 100/1,200/1, and 300/1, and then mixed so that the number of each peptides with respect to the number of gold nanoparticles became 2000, thereby to prepare multifunctional metal nanoparticles.

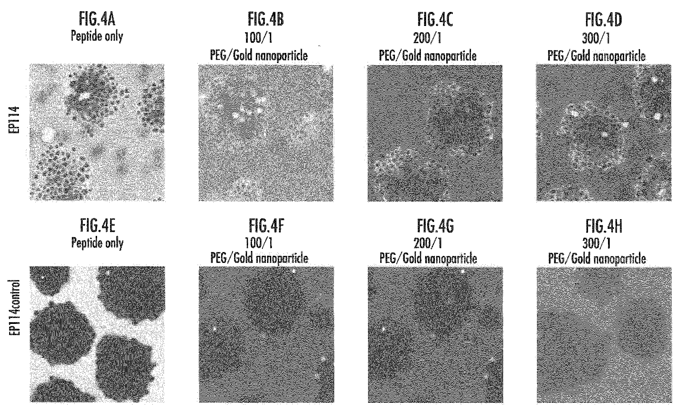

As shown in FIG. 4A, FIG. 4B, FIG. 4C, FIG. 4D, FIG. 4E, FIG. 4F, FIG. 4G and FIG. 4H, the cells were confirmed to be stained through the binding of the multifunctional metal nanoparticles to EpCAM on the cell surface. In the case of using the EpCAM-binding peptides as the biologically functional molecules, the cells can be detected under a fluorescence microscope at levels equivalent among any of the number of PEG molecules : number of gold nanoparticles ratio values of 100/1 (FIG. 4B, FIG. 4F), 200/1 (FIG. 4C, FIG. 4G), and 300/1 (FIG. 4D, FIG. 4H). Furthermore, (FIG. 4A) and (FIG. 4E) shows the results of reacting the peptide alone with the cell instead of using gold nanoparticles covered with peptide.

As shown above, the metallic nanostructure covered with peptides or antibodies capable of binding to surface antigens (e.g., EpCAM) expressed in cancer cells, as the biologically functional molecules, can achieve specific staining of the cells.

### [Example 3] Cell staining using multifunctional metal nanostructure covered with plectin binding peptides

Plectin is recently reported as a biomarker for pancreatic cancer, and the localization is detected by the peptides binding to plectin (Non-Patent Literatures 3 and 4). Therefore, the plectin binding peptides were subjected to bonding evaluation test of the gold nanoparticles surface-modified with peptide.

A colloidal solution of gold nanoparticles, i-colloid Au15 (manufactured by IMRA America, Inc., USA) and FITC-PEG-SH (manufactured by NANOCOS) were prepared and covered such that the ratio value of number of PEG molecules : number of gold nanoparticles was 200/1. The modification rate was 50%.

Next, plectin binding peptides which was amidated at the C-terminal was prepared and covered such that the ratio value of the number of peptides and the number of gold particles was 600/1. Here, two types of plectin binding peptides, one provided with an amino acid sequence linker and one without the amino acid sequence linker, were prepared.

Plectin binding peptide sequence
With a linker: KTLLPTPGGC (SEQ ID NO.3)
No linker: KTLLPTP (SEQ ID NO. 4)
By covering with the peptides at the above described ratio, approximately 50% portion which was not covered with PEG was covered, and almost all of the surface of the gold nanoparticles became a covered state.

The bonding evaluation test of the gold nanoparticles modified by plectin binding peptides was performed by using MIAPaCa2 in which plectin localizes on the cell surface.

MIAPaCa2 was seeded at a concentration of 2.5 × 10⁴ cell/well on a Bio Coat Poly-D-Lisine 8-well slide (manufactured by Becton, Dickinson and Company), and cultured under the condition of 5% CO₂ at 37°C for 4 hours.

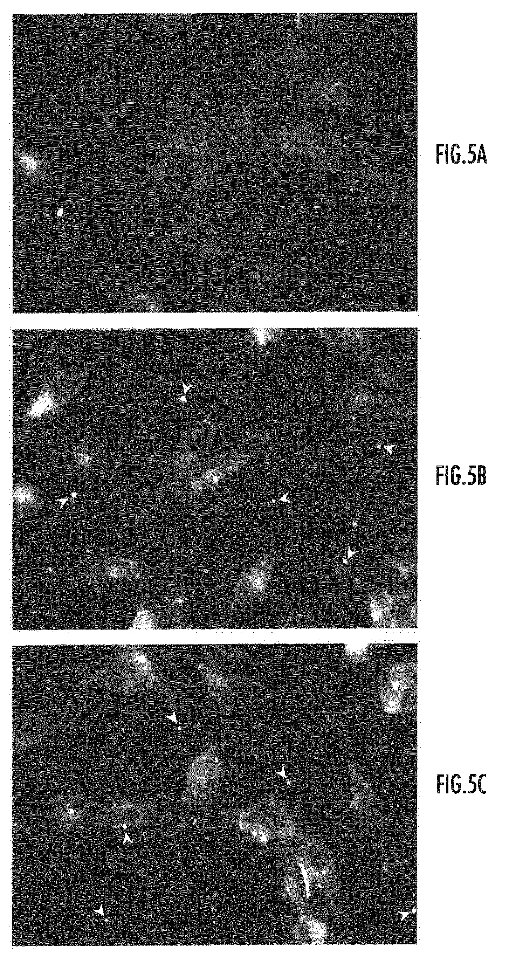

Using 200µl of 4% paraformaldehyde (PFA) in phosphate buffered solution (PBS), after being fixed at room temperature for 10 minutes, it was washed twice with PBS containing 250µl of 1% bovine serum albumin (BSA) (hereinafter referred to as 1% BSA / PBS). Furthermore, it was subjected for blocking by being stilled in the 1% BSA / PBS at room temperature for 1 hour, then added with 200µl of gold nanoparticles modified by 20nM plectin binding peptides. After being stilled for 3 hours, it was washed twice by 250µl of 1% BSA / PBS, and was sealed by using a Prolong gold antifade reagent with DAPI special packaging (manufactured by Invitrogen Corporation).
The specimen was observed using a dark field microscope (DMLP Polarizing microscope, manufactured by LEICA, Leica Microsystems, K.K.) installed with HRA nano imaging adapter (manufactured by CytoVIVA, Inc.). The results are shown in Figure 5.

FIG. 5A shows the result of using gold nanoparticles covered with PEG only, FIG. 5B shows the result of using gold nanoparticles covered with plectin binding peptides with a linker, and FIG. 5C shows the result of using gold nanoparticles covered with plectin binding peptides without a linker. As clearly shown from these micrographs, scattered light signals by the gold nanoparticles are observed on the cell surface of gold nanoparticle of FIG. 5B and FIG. 5C modified by peptides compared to gold nanoparticles of FIG. 5A which was not modified by plectin binding peptides. Moreover, strong signals are observed between the cells as indicated by arrows. It is reported that plectin is emitted outside the cell, and it can be conceived that the plotline outside the cell is detected.

### [Example 4]

We have also examined the application of the metal nanostructures modified in the present invention to flow cytometry. The fluorescence intensity was measured for the gold nanoparticles only, gold nanoparticles covered with PEG, gold nanoparticles used in Example 3 covered with PEG with FITC bonded, FITC-PEG-SH, and in addition to this, those further bonded with peptide.

The samples were diluted with purified water to adjust to OD₅₂₀ = 1, by using a fluorescence spectrophotometer FP-6500 (manufactured by JASCO Corporation), the fluorescence was measured under the measuring conditions = Response: 1 sec, Band width (Ex): 5nm, Band width (Em): 5nm, Sensitivity: medium. Excitation wavelength was set at 495nm, and the measurement fluorescence wavelength was set at 519nm.

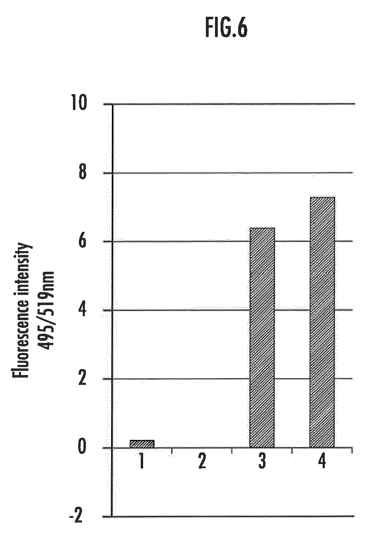

As shown in FIG. 6, almost no fluorescence was observed for (1) blank (purified water) and (2) those covered with PEG without FITC binding, while (3) those covered with FITC-PEG-SH and (4) those covered with FITC-PEG-SH and further covered with peptide were observed to show significantly enhanced fluorescence intensity.

By using PEG bonded with FITC as the colloid-stabilizing functional molecules, it is able to observe fluorescence by modifying with any biologically functional molecules. In other words, the detection using FACS or fluorescence microscope becomes possible by using colloid-stabilizing functional molecules which are marked with fluorescence dye or the like without marking each of the biologically functional molecules such as the applied peptide, the adapter, or the like.

Further, as shown in Example 3, since the light scattered by the gold nanoparticles can be observed, it is possible to confirm the binding by also using a detector that detects scattered light such as the dark field microscope or the like.

Biologically functional molecules capable of binding to EpCAM, plectin, or other proteins, for example, expressed on cell surface are arbitrarily selected. Therefore, the method of the present invention can be used in diagnosis or treatment targeting not only cancer cells but also various cells.

As shown above, the multifunctional metallic nanostructure of the present invention can be bound with, for example, arbitrary antibodies or aptamers according to research, diagnostic, or therapeutic purposes and thereby can be utilized in various uses.

## Claims

1. A multifunctional metallic nanostructure comprising a metallic nanostructure having a surface covered with:
at least one or more types of colloid-stabilizing functional molecules which cover 30 to 90% of the surface of the metallic nanostructure; and
at least one or more types of biologically functional molecules having a terminal amino acid.

2. The multifunctional metallic nanostructure according to claim 1, wherein
the colloid-stabilizing functional molecules include a compound represented by a following general formula (1):
[Formula 1] -(CH₂-CH₂-O)ₙ- (1)
wherein n represents an integer of 1 or larger.

3. The multifunctional metallic nanostructure according to claim 2, wherein
the compound of the general formula (1) is polyethylene glycol or a derivative thereof.

4. The multifunctional metallic nanostructure according to claim 1, wherein
the colloid-stabilizing functional molecules each have a functional group having thiol (- SH) or disulfide (- S - S -) at least at one end thereof.

5. The multifunctional metallic nanostructure according to claim 4, wherein
the colloid-stabilizing functional molecules each have
a functional group having thiol (- SH) or disulfide (- S - S -) at one end, and
at least any one of a methoxy group, an amino group, a carboxy group, an acyl group, an azo group, and a carbonyl group at the other end.

6. The multifunctional metallic nanostructure according to claim 1, wherein
the metallic nanostructure is a metal nanoparticle, wherein
the metal nanoparticle is a noble metal nanoparticle or a noble metal-containing alloy nanoparticle.

7. The multifunctional metallic nanostructure according to claim 6, wherein
the metal nanoparticle is a gold nanoparticle or a gold-containing alloy nanoparticle.

8. The multifunctional metallic nanostructure according to claim 1, wherein
the biologically functional molecules comprise peptide.

9. A dispersion of a multifunctional metallic nanostructure dispersed in a liquid, wherein
the dispersion comprises a multifunctional metallic nanostructure according to claim 1.

10. A freeze-dried product comprising a multifunctional metallic nanostructure, wherein
the dispersion according to claim 9 is frozen.

11. A composition for diagnosis and/or treatment comprising a multifunctional metallic nanostructure, wherein
the composition comprises the multifunctional metallic nanostructure according to claim 1.

12. A method for manufacturing a multifunctional metallic nanostructure, comprising the steps of:
providing a metallic nanostructure dispersed in water or an electrolyte solution;
covering 30 to 90% of a surface of the metallic nanostructure with colloid-stabilizing functional molecules, by monitoring an amount of surface covering of the metallic nanostructure with measurement of a physical quantity; and
covering the surface of the metallic nanostructure with one or more types of biologically functional molecules.

13. The method for manufacturing a multifunctional metallic nanostructure according to claim 12, wherein
a dispersion of the metallic nanostructure dispersed in water or an electrolyte solution has an electric conductivity of approximately 25 µS/cm or lower.

14. The method for manufacturing a multifunctional metallic nanostructure according to claim 12, wherein
the one or more types of biologically functional molecules are N types (wherein N represents an integer) of biologically functional molecules, the method further comprising
individual steps of using a first biologically functional molecules to a (N - 1)th biologically functional molecules as the biologically functional molecules to each partially cover the surface of the metallic nanostructure in order so as not to occupy a whole of an effective surface area thereof,
while adjusting the amount of surface covering of the metallic nanostructure in each of the individual steps by the measurement of the physical quantity, whereafter
the metallic nanostructure is surface-covered with the Nth biologically functional molecules until an effective region on the surface of the metallic nanostructure becomes saturated.

15. The method for manufacturing a multifunctional metallic nanostructure according to claim 12, further comprising the step of
removing redundant molecules unbound with the metallic nanostructure after each of the individual steps.

16. The method for manufacturing a multifunctional metallic nanostructure according to claim 15, wherein
the step of removing redundant molecules involves centrifugation or dialysis.

17. A kit for manufacturing a multifunctional metallic nanostructure according to claim 1, comprising:
a metallic nanostructure partially covered with at least one or more types of colloid-stabilizing functional molecules; and
a buffer solution for covering with biologically functional molecules.


SEQUENCE LISTING

<110>  IMURA AMERICA, INC.
       Japanese Foundation for Cancer Research

<120>  Multifunctional metallic nanostructure and method for
       manufactureing the same

<130>  PCT140179

<160>  4

<170>  PatentIn version 3.5

<210>  1
<211>  15
<212>  PRT
<213>  Artificial Sequence

<220>
<223>  EpCAM binding peptide

<400>  1

Lys His Leu Gln Cys Val Arg Asn Ile Cys Trp Ser Gly Gly Lys
1               5                   10                  15


<210>  2
<211>  12
<212>  PRT
<213>  Artificial Sequence

<220>
<223>  EpCAM binding control

<400>  2

Lys His Ala Gln Cys Val Arg Asn Ile Cys Trp Ser
1               5                   10


<210>  3
<211>  10
<212>  PRT
<213>  Artificial Sequence

<220>
<223>  Plectin binding peptide w linker

<400>  3

Lys Thr Leu Leu Pro Thr Pro Gly Gly Cys
1               5                   10


<210>  4
<211>  7
<212>  PRT
<213>  Artificial Sequence

<220>
<223>  Plectin binding peptide w/o linker

<400>  4

Lys Thr Leu Leu Pro Thr Pro
1               5